(19) Europäisches Patentamt
European Patent Office
Office européen des brevets

(11) **EP 1 862 470 A1**

(12) **EUROPEAN PATENT APPLICATION**
published in accordance with Art. 158(3) EPC

(43) Date of publication:
**05.12.2007 Bulletin 2007/49**

(51) Int Cl.:
*C07K 7/00* (2006.01)        *A61K 47/10* (2006.01)
*A61K 9/127* (2006.01)        *A61K 31/711* (2006.01)
*A61K 45/00* (2006.01)        *A61K 47/24* (2006.01)
*A61K 47/42* (2006.01)        *A61K 48/00* (2006.01)
*A61P 35/00* (2006.01)

(21) Application number: **06714507.8**

(22) Date of filing: **24.02.2006**

(86) International application number:
**PCT/JP2006/303368**

(87) International publication number:
**WO 2006/090813 (31.08.2006 Gazette 2006/35)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **25.02.2005 JP 2005050708**

(71) Applicants:
• **National University Corporation Hokkaido University**
**Sapporo-shi, Hokkaido 0600808 (JP)**
• **University of Tsukuba**
**Tsukuba-shi, Ibaraki 305-8577 (JP)**

(72) Inventors:
• **AKITA, Hidetaka**
**Tsububa-shi, Ibaraki, 3058577 (JP)**

• **HATAKEYAMA, Hiroto**
**Tsububa-shi, Ibaraki, 3058577 (JP)**
• **NAGASAKI, Yukio**
**Tsububa-shi, Ibaraki, 3058577 (JP)**
• **KIKUCHI, Hiroshi,**
**DAIICHI PHARMACEUTICAL CO. Ltd.**
**Tokyo 134-8630 (JP)**
• **KOBAYASHI, Hideo,**
**DAIICHI PHARMACEUTICAL CO. Ltd.**
**Tokyo 134-8630 (JP)**
• **HARASHIMA, Hideyoshi**
**Tsukuba-shi, Ibaraki, 3058577 (JP)**
• **OISHI, Motoi**
**Tsukuba-shi, Ibaraki, 3058577 (JP)**

(74) Representative: **Polypatent**
**An den Gärten 7**
**51491 Overath (DE)**

(54) **ELEMENT RETAINED IN BLOOD SHOWING DEGRADABILITY SELECTIVELY IN TUMOR TISSUE**

(57)    A phospholipid derivative useful for the preparation of liposomes for efficient uptake of an antitumor agent or a gene intracellularly by a target tumor cell, which comprises a residue of an alcohol compound and a residue of a phospholipid, and comprising a peptide between the residue of an alcohol compound and the residue of a phospholipid, and wherein (a) the alcohol compound is an alcohol compound selected from poly (alkylene glycols) and the like, (b) the phospholipid is a phospholipid selected from phosphatidylethanolamines, phosphatidylcholines, phosphatidylserines and the like, and (c) the peptide is a peptide comprising a substrate peptide that can serve as a substrate of a matrix metalloproteinase, provided that one amino acid residue or an oligopeptide containing 2 to 8 amino acid residues may bind to one or both ends of the substrate peptide.

**EP 1 862 470 A1**

**Description**

Technical Field

The present invention relates to a phospholipid derivative that is bound with poly(alkylene glycol) or the like via a peptide and useful as a component lipid of lipid membrane structures such as liposomes, and to a blood retainable device comprising said phospholipid derivative and exhibiting selective degradability in a tumor tissues.

Background Art

As a means for transporting a medicament specifically to a pathological lesion, methods of encapsulating a medicament in liposomes have been proposed. In particular, in the field of therapeutic treatments of malignant tumors, many reports have been made as for effectiveness of liposomes encapsulating an antitumor agent. Further, a multifunctional envelope-type nano device (MEND, henceforth sometimes abbreviated as "MEND" in the specification) has been proposed. This structure can be used as a drug delivery system for delivering a gene or the like selectively into particular cells, and is known to be useful for, for example, gene therapy of tumors and the like.
However, microparticle carriers such as liposomes and the aforementioned MEND have problems that they have poor blood retainability when intravenously administered and are likely captured by tissues of reticuloendothelial system in the liver, spleen and the like. Moreover, these microparticle carriers also have problems that they may cause leakage of an encapsulated substance or aggregation of microparticles. These problems are serious obstacles to targeting therapies for delivering a medicament or gene to a target organ or target cells by using liposomes encapsulating the medicament or the aforementioned MEND encapsulating the gene.
As one of means for avoiding the aforementioned problems, a means for modifying surfaces of microparticle carriers such as liposomes with a poly(alkylene glycol) (polyethylene glycol (PEG) and the like) has been proposed (Biochim. Biophys. Acta, 1066, pp.29-36, 1991; FEBS Lett., 268, pp.235-237, 1990; Biochim. Biophys. Acta, 1029, pp. 91-97, 1990). This means is based on the findings that, when a hydration layer constituted by PEG covers microparticle carriers such as liposomes, opsoninization such as adsorption of serum proteins is suppressed, and as a result, phagocytosis by macrophages and uptake by the reticuloendothelial system can be avoided. For this purpose, phospholipids bound with a poly(alkylene glycol) have been proposed, and it is known that surfaces of liposomes can be modified with a poly(alkylene glycol) by using the phospholipids. Further, PEG-modified liposomes of which particle diameters are controlled to be 100 to 200 nm are almost selectively accumulated in a solid tumor due to the EPR (Enhanced Permeability and Retention) effect, and maintained in the tumor over a long period of time without being recovered again in the blood (as for the EPR effect, see, Cancer Res., 46, pp.6387-6392, 1986).
However, when the surfaces of microparticle carriers such as liposomes are modified with a poly(alkylene glycol), it is known that another problem arises in that the microparticle carriers become hard to be taken up intracellularly by target cells, although blood retainability is improved. Particularly in a targeting therapy where an antitumor agent or a gene is delivered specifically to target tumor cells by using drug-encapsulating liposomes or a gene-encapsulating MEND, this problem causes a serious problem that therapeutic effect cannot be attained to an expected degree, and side reactions, caused by the antitumor agent or the gene remained undelivered to the target tumor cells, cannot be sufficiently avoided,.
Tumor cells decompose and reconstruct extracellular matrix (ECM) in processes of proliferation, infiltration, and metastasis, and in these processes, matrix metalloproteinases (MMPs) are known to play important roles (Cell, 91, pp.439-442, 1997; APMIS, 107, pp.137-143, 1999). As for MMPs, 20 or more families have been identified, and they are classified into the class of secretion type and that of membrane type which exist on cell membranes. MMPs have a function of decomposing ECMs such as collagen. Peptide sequences specifically decomposed and cleaved by MMPs have been revealed by recent studies (Nature Biotechnology, 19, pp.661-667, 2001).
A peptide-bound phospholipid usable as a component lipid of liposomes is also known (Japanese Patent Unexamined Publication based on international patent application (KOHYO) No. 2003-513009). The aforementioned patent document describes that the peptide of the phospholipid may be modified with PEG (polyethylene glycol) or the like (paragraph [0016] of the aforementioned patent document). When the peptide of this phospholipid is cleaved by a peptidase, liposomes containing said lipid as a component lipid become unstable, and encapsulated substance is released at that site. Therefore, the encapsulated substance can be specifically delivered to peptidase-secreting cells. It is also taught that a matrix metalloproteinase can be used as the aforementioned peptidase, and liposomes can be used for targeting tumor cells (paragraphs [0021] and [0042] of the aforementioned patent document). However, efficiency of delivery for a medicament or a gene achieved by liposomes using this phospholipid is not fully satisfactory.

Disclosure of the Invention

Object to be Achieved by the Invention

For delivering an antitumor agent or a gene for gene therapy of malignant tumors into target tumor cells by using a lipid membrane structure such as liposome of which surface is modified with a poly(alkylene glycol) or the like and MEND as a microparticle carrier, an object of the present invention is to provide a means for achieving efficient uptake of the antitumor agent or gene intracellularly by the target tumor cells. More specifically, the object of the present invention is to provide a phospholipid derivative usable for preparing the aforementioned lipid membrane structure and utilizable as a means for efficiently achieving uptake of an antitumor agent or a gene intracellularly by target tumor cells.

Means for Achieving the Object

The inventors of the present invention conducted various researches to achieve the aforementioned object. When a particular phospholipid modified with a poly(alkylene glycol) or the like, in which an oligopeptide hydrolysable with a matrix metalloproteinase is inserted between a modification moiety such as poly(alkylene glycol) and a phospholipid moiety, is used to prepare a lipid membrane structure such as liposome and MEND, and the lipid membrane structure is used as a microparticle carrier for an antitumor agent, gene or the like, superior blood retainability of the liposome or MEND is maintained in blood due to the presence of the modification moiety such as poly(alkylene glycol), whereas the oligopeptide moiety in the liposome or MEND having reached to a target tumor tissue is hydrolyzed by a matrix metalloproteinase to dissociate the modification moiety such as poly(alkylene glycol). As a result, according to a method wherein the stability of the liposome or MEND is reduced, from which the modification moiety such as poly(alkylene glycol) is dissociated, and the liposome or MEND can no longer maintain their structures, thereby they release the antitumor agent retained in the lipid membrane structure to the extracellular space of the target tumor cells, or according to another method wherein the liposome or MEND changes to those wherein the modification moiety such as poly(alkylene glycol) is dissociated, and the resulting lipid membrane structure, per se, is efficiently taken up by the target tumor cells, or according to a combination of these methods, the inventors found that the antitumor agent or gene was successfully delivered into target tumor cells at an extremely high introduction rate. It was also found that extremely high antitumor effect was achievable by efficiently delivering the antitumor agent or gene to the target cells as described above. The present invention was accomplished on the basis of the aforementioned findings.

The present invention thus provides a phospholipid derivative comprising a residue of an alcohol compound and a residue of a phospholipid, and comprising a peptide between the residue of an alcohol compound and the residue of a phospholipid, wherein

    (a) the alcohol compound is selected from the group consisting of poly(alkylene glycols), glycerins, and polyglycerins,
    (b) the phospholipid is selected from the group consisting of phosphatidylethanolamines, phosphatidylcholines, phosphatidylserines, phosphatidylinositols, phosphatidylglycerols, cardiolipins, sphingomyelins, ceramide phosphorylethanolamines, ceramide phosphorylglycerols, ceramide phosphorylglycerol phosphates, 1,2-dimyristoyl-1,2-deoxyphosphatidylcholines, plasmalogens and phosphatidic acids, and
    (c) the peptide comprises a substrate peptide that can serve as a substrate of a matrix metalloproteinase (provided that one amino acid residue or an oligopeptide containing 2 to 8 amino acid residues may bind to one or both ends of the substrate peptide).

According to preferred embodiments of the present invention, there are provided the aforementioned phospholipid derivative, wherein the alcohol compound is a poly(alkylene glycol), the phospholipid is a phosphatidylethanolamine, and the peptide is a peptide containing Val-Pro-Leu-Ser-Leu-Tyr-Ser-Gly; the aforementioned phospholipid derivative, wherein the alcohol compound is a poly(ethylene glycol), the phospholipid is dioleoylphosphatidylethanolamine, and the peptide contains Gly-Gly-Gly as a linker; and the aforementioned phospholipid derivative, wherein the peptide is Gly-Gly-Gly-Val-Pro-Leu-Ser-Leu-Tyr-Ser-Gly-Gly-Gly-Gly.

From another aspect, the present invention provides a lipid membrane structure comprising the aforementioned phospholipid derivative as a component lipid. According to preferred embodiments of this invention, there are provided the aforementioned lipid membrane structure, which comprises an antitumor agent or a gene for gene therapy of a malignant tumor; and the aforementioned lipid membrane structure, which is a liposome.

From a still further aspect, the present invention provides a pharmaceutical composition for therapeutic treatment of a malignant tumor, which contains the aforementioned lipid membrane structure comprising an antitumor agent or a gene for gene therapy of the malignant tumor.

In addition to these inventions, the present invention provides a method for therapeutic treatment of a malignant tumor, which comprises the step of administrating the aforementioned lipid membrane structure comprising an antitumor agent or a gene for gene therapy of the malignant tumor to a mammal including human.

Brief Description of the Drawings

[Fig. 1] Schematic drawings showing structural classification of amphipathic lipid molecules and structures of lipid aggregates.
[Fig. 2] A graph showing relationship between addition rate of DOPE-PEG and particle diameter of liposomes. The vertical axis indicates the particle diameter (nm).
[Fig. 3] A graph showing relationship between addition rate of PEG-peptide-DOPE and particle diameter of liposomes. The vertical axis indicates the particle diameter (nm).
[Fig. 4] A graph showing results of evaluation of PEG cleavage by MMP-2 performed by using DOPE liposomes containing PEG-peptide-DOPE at a rate of 10%. The vertical axis indicates particle diameter (nm).
[Fig. 5] A graph showing results of expression of a gene attained by introducing the gene into HT1080 cells using MENDs comprising PEG-peptide-DOPE.
[Fig. 6] A graph showing results of expression of a gene attained by introducing the gene into HEK293 cells using MENDs comprising PEG-peptide-DOPE.
[Fig. 7] A graph showing results of expression of a gene attained by introducing the gene into HT1080 cells using MENDs comprising PEG-peptide-DOPE. In the graph, PEG-MEND means the result of PEG-DSPE modification group, and PPD-MEND means the result of the PEG-peptide-DOPE modification group.

Best Mode for Carrying out the Invention

The phospholipid derivative of the present invention is a phospholipid derivative comprising a residue of an alcohol compound and a residue of a phospholipid, and comprising a peptide between the residue of an alcohol compound and the residue of a phospholipid, characterized in that

(a) the alcohol compound is selected from the group consisting of poly(alkylene glycols), glycerins, and polyglycerins,
(b) the phospholipid is selected from the group consisting of phosphatidylethanolamines, phosphatidylcholines, phosphatidylserines, phosphatidylinositols, phosphatidylglycerols, cardiolipins, sphingomyelins, ceramide phosphorylethanolamines, ceramide phosphorylglycerols, ceramide phosphorylglycerol phosphates, 1,2-dimyristoyl-1,2-deoxyphosphatidylcholines, plasmalogens and phosphatidic acids, and
(c) the peptide is a peptide comprising a substrate peptide that can serve as a substrate of a matrix metalloproteinase (provided that one amino acid residue or an oligopeptide containing 2 to 8 amino acid residues may bind to one or both ends of the substrate peptide).

As the alcohol compound, poly(alkylene glycols) such as polyethylene glycol and polypropylene glycol, glycerins such as glycerin and glycerin ester, and polyglycerins such as diglycerin, triglycerin, tetraglycerin, pentaglycerin, hexaglycerine, heptaglycerin, and octaglycerin can be used. Among them, poly(alkylene glycols) are preferred, and particularly preferred is polyethylene glycol. When polyethylene glycol is used, the molecular weight thereof is not particularly limited, and can be suitably chosen by those skilled in the art for imparting desired characteristics such as retainability in blood to lipid membrane structures.
The residue of an alcohol compound means a group obtained by eliminating an appropriate atom such as hydrogen atom, or an appropriate functional group such as hydroxyl group or a halogen atom from an alcohol compound or a chemically modified alcohol compound, preferably such a monovalent group. Examples include, for example, a residue obtained by eliminating hydrogen atom from hydroxyl group of an alcohol compound, a residue obtained by eliminating hydrogen atom binding to a carbon atom of an alcohol compound, a residue obtained by introducing a functional group comprising carboxyl group to an end of an alcohol compound and eliminating hydroxyl group or hydrogen atom from the carboxyl group, and the like. However, examples are not limited to those mentioned above.
As the phospholipid, phosphatidylethanolamines, phosphatidylcholines, phosphatidylserines, phosphatidylinositols, phosphatidylglycerols, cardiolipins, sphingomyelins, ceramide phosphorylethanolamines, ceramide phosphorylglycerols, ceramide phosphorylglycerol phosphates, 1,2-dimyristoyl-1,2-deoxyphosphatidylcholines, plasmalogens and phosphatidic acids can be used, and the aliphatic acid residue in these phospholipids is not particularly limited. For example, a phospholipid having one or two saturated or unsaturated aliphatic acid residues each having about 12 to 20 carbon atoms can be used, and specifically, a phospholipid having one or two acyl groups derived from an aliphatic acid such as lauric acid, myristic acid, palmitic acid, stearic acid, oleic acid, and linoleic acid can be used. Among those mentioned above, phosphatidylethanolamines are preferred, and particularly preferred is dioleoylphosphatidylethanolamine (DOPE).

The residue of a phospholipid means a group obtained by eliminating an appropriate atom such as hydrogen atom, or an appropriate functional group such as hydroxyl group or a halogen atom from any of the aforementioned phospholipid or a chemically modified phospholipid, preferably such a monovalent group. Examples include, for example, a residue obtained by eliminating hydrogen atom from hydroxyl group of phosphoric acid moiety of a phospholipid, a residue obtained by eliminating hydroxyl group of phosphoric acid moiety of a phospholipid, a residue obtained by eliminating hydrogen atom binding to a carbon atom of a phospholipid, a residue obtained by introducing a functional group comprising carboxyl group into phosphoric acid moiety of a phospholipid and eliminating hydroxyl group or hydrogen atom from the carboxyl group, and the like. However, examples are not limited to those mentioned above.

The peptide contained between the residue of an alcohol compound and the residue of a phospholipid contains at least one substrate peptide that can serve as a substrate of a matrix metalloproteinase. As the matrix metalloproteinase (MMP), for example, MMP-1 (interstitial collagenase), MMP-2 (gelatinase A), MMP-3, MMP-7, MMP-9 (gelatinase B), and the like are known, and a substrate peptide that can serve as a substrate of one or more kinds of matrix metalloproteinases among those mentioned above can be used. For matrix metalloproteinases, for example, "Molecular mechanism of cancer metastasis", Ed. by Tsuruo T., pp.92-107, Medical View Co., Ltd., published on 1993, and the like can be referred to.

As for the substrate peptide that can serve as a substrate of a matrix metalloproteinase, for example, the matrix metalloproteinases of particular types and substrate peptides specifically recognized thereby are explained in Non-patent document 1 (Nature Biotechnology, 19, pp.661-667, 2001) mentioned above. Therefore, by referring to this publication, a substrate peptide specifically cleaved by a particular type of matrix metalloproteinase can be chosen. For example, Val-Pro-Leu-Ser-Leu-Tyr-Ser-Gly is known as a specific substrate for MMP-9, and it is preferable to use the aforementioned octapeptide as a substrate peptide that can serve as a substrate of MMP-9. The entire disclosure of Non-patent document 1 mentioned above is incorporated herein by reference.

Specifically, examples of the substrate peptide that can serve as a substrate of a matrix metalloproteinase include Gly-Pro-Gln-Gly-Ile-Ala-Gly-Gln, Gly-Pro-Gln-Gly-Ile-Ala-Gly-Gln, Val-Pro-Met-Ser-Met-Arg-Gly-Gly, Ile-Pro-Val-Ser-Leu-Arg-Ser-Gly, Arg-Pro-Phe-Ser-Met-Ile-Met-Gly, Val-Pro-Leu-Ser-Leu-Thr-Met-Gly, Ile-Pro-Glu-Ser-Leu-Arg-Ala-Gly, Arg-His-Asp, Arg-Pro-Lys-Pro-Val-Glu-Nva-Trp-Arg-Lys, Arg-Pro-Lys-Pro-Tyr-Ala-Nva-Trp-Met-Lys, Pro-Gln-Gly-Ile-Ala-Gly-Gln-Arg, Pro-Leu-Gly-Ile-Ala-Gly-Arg, Gly-Pro-Leu-Gly-Pro, Gly-Pro-Leu-Gly-Pro, and the like.

The peptide contained between the residue of an alcohol compound and the residue of a phospholipid may contain, besides the substrate peptide, a linker consisting of one amino acid residue, or a linker consisting of an oligopeptide containing 2 to 8 amino acid residues as a linker involved in the bond or bonds with the residue of an alcohol compound and/or the residue of a phospholipid. The amino acid residue or oligopeptide as the linker may bind to the both ends of the substrate peptide, or may bind only to one end of the substrate peptide. When the linker binds to the both ends of the substrate peptide, two linkers may be the same or different. The substrate peptide may directly bind to the residue of an alcohol compound and/or the residue of a phospholipid without a linker. It is preferred that the phospholipid derivative of the present invention contains a linker, it is more preferred that the same or different linkers bind to the both ends of the substrate peptide, and it is still more preferred that two of the linkers are the same or different linkers each consisting of an oligopeptide containing 2 to 8 amino acid residues.

Types of one amino acid residue usable as the linker, and amino acid residues constituting an oligopeptide usable as the linker are not particularly limited, and one amino acid residue of an arbitrary type, or an arbitrary oligopeptide containing 2 to 8 of the same or different amino acid residues of arbitrary types can be used. Examples of the oligopeptide usable as the linker include, for example, -Leu-Gly-, - Tyr-Gly-, -Phe-Gly-, -Gly-Phe-Gly-, -Gly-Gly-Phe-Gly-, -Gly-Phe-Gly-Gly-, -Phe-Gly-Gly-Gly-,- Phe-Phe-Gly-Gly-, -Gly-Gly-Gly-Phe-Gly-, -Gly-Gly-Phe-Phe-, -Gly-Gly-Gly-Gly-Gly-Gly-, -Phe-Phe-, -Ala-Gly-, -Pro-Gly-, -Gly-Gly-Gly-Phe-, -Gly-, -D-Phe-Gly-, - Gly-Phe-, -Ser-Gly-, -Gly-Gly-, -Gly-Gly-Gly-, -Gly-Gly-Gly-Gly-, -Gly-Gly-Leu-Gly-, - Gly-Gly-Tyr-Gly-, -Gly-Gly-Val-Leu-, -Gly-Gly-Leu-Leu-, -Gly-Gly-Phe-Leu-, -Gly-Gly-Tyr-Leu-, -Gly-Gly-Val-Gln-, -Gly-Gly-Leu-Gln-, -Gly-Gly-Ile-Gln, -Gly-Gly-Phe-Gln-, - Gly-Gly-Tyr-Gln-, -Gly-Gly-Trp-Gln-, -Gly-Gly-Leu-Ser-, -Gly-Gly-Phe-Ser-, -Gly-Gly-Tyr-Ser-, -Gly-Gly-Val-Thr-, -Gly-Gly-Leu-Thr-, -Gly-Gly-Phe-Thr-, -Gly-Gly-Tyr-Thr-, -Gly-Gly-Trp-Thr-, -Gly-Gly-Val-Met-, -Gly-Gly-Leu-Met-, -Gly-Gly-Ile-Met-, -Gly-Gly-Phe-Met-, -Gly-Gly-Tyr-Met-, -Gly-Gly-Val-Cit-, -Gly-Gly-Leu-Cit-, -Gly-Gly-Phe-Cit-, -Gly-Gly-Tyr-Cit-, -Gly-Gly-Trp-Cit-, -Gly-Gly-Gly-Asn-, -Gly-Gly-Ala-Asn-, -Gly-Gly-Val-Asn-, -Gly-Gly-Leu-Asn-, -Gly-Gly-Ile-Asn-, -Gly-Gly-Gln-Asn-, -Gly-Gly-Thr-Asn-, -Gly-Gly-Phe-Asn-, -Gly-Gly-Tyr-Asn-, -Gly-Gly-Met-Asn-, -Gly-Gly-Pro-Asn-, -Gly-Gly-Cit-Asn-, -Gly-Gly-Trp-Gly-, -Gly-Gly-Ser-Asn-, -Gly-Gly-Pro-Ala-, -Gly-Gly-Pro-Val-, -Gly-Gly-Pro-Leu-, -Gly-Gly-Pro-Ile-, -Gly-Gly-Pro-Gln-, -Gly-Gly-Pro-Ser-, -Gly-Gly-Pro-Tyr-, -Gly-Gly-Pro-Met-, -Gly-Gly-Met-Pro-, -Gly-Gly-Pro-Pro-, -Gly-Gly-Pro-Cit-, -Gly-Gly-Ile-Leu-, -Gly-Gly-Ile-Cit-, and the like, but examples are not limited to those mentioned above. Among them, -Gly-Gly- or -Gly-Gly-Gly- is preferred as the linker.

Although the method for preparing the phospholipid derivative of the present invention is not particularly limited, the derivative can be generally prepared by binding the alcohol compound and the peptide compound as the partial structures of the aforementioned phospholipid derivative, and binding a phospholipid compound to the peptide end of the resulting peptide-bound alcohol compound.

The binding of the alcohol compound and the peptide compound is generally attained by reacting amino group or carboxyl group of the peptide end of the peptide compound and a reactive functional group (for example, hydroxyl group, carboxyl group, ester group and the like) of the alcohol compound. Although hydroxyl group already existing in the alcohol compound may be used as the reactive functional group, carboxyl group may be introduced into the alcohol compound by, for example, a method of oxidizing hydroxyl group of the alcohol compound to carboxyl group, or a method of introducing a functional group containing carboxyl group into the alcohol compound, the carboxyl group may be further esterified as required, and the carboxyl group or esterified carboxyl group may be used as the reactive functional group.

Typically, by reacting a functional group of the alcohol compound such as carboxyl group or ester group and amino group of the peptide compound to form an amide bond, a peptide-bound alcohol compound can be prepared. For this reaction, various methods such as the acid halide method, the active ester method and the acid anhydride method can be used.

In the acid halide method, by treating an alcohol compound having carboxyl group with a halogenating agent in an inert solvent to prepare an acid halide, and reacting the resulting acid halide with amino group of a peptide compound, the objective compound can be obtained. Type of the solvent used in the reaction for producing an acid halide is not particularly limited, and any solvents that dissolve the starting materials without inhibiting the reaction may be used. Preferred are, for example, ethers such as diethyl ether, tetrahydrofuran and dioxane, amides such as dimethylformamide, dimethylacetamide and hexamethylphosphoric acid triamide, halogenated hydrocarbons such as dichloromethane, chloroform and 1,2-dichloroethane, nitriles such as acetonitrile and propionitrile, esters such as ethyl formate and ethyl acetate, and mixed solvents of these solvents. Examples of the halogenating agent include, for example, thionyl halides such as thionyl chloride, thionyl bromide and thionyl iodide, sulfuryl halides such as sulfuryl chloride, sulfuryl bromide and sulfuryl iodide, phosphorus trihalides such as phosphorus trichloride, phosphorus tribromide and phosphorus triiodide, phosphorus pentahalides such as phosphorus pentachloride, phosphorous pentabromide and phosphorus pentaiodide, phosphorus oxyhalides such as phosphorus oxychloride, phosphorus oxybromide and phosphorus oxyiodide, oxalyl halides such as oxalyl chloride and oxalyl bromide, and the like. The reaction may be performed at a temperature of from 0°C to the reflux temperature of the solvent, preferably at a temperature of from room temperature to the reflux temperature of the solvent.

The solvent used for the reaction of the resulting acid halide and amino group of the peptide compound is not particularly limited so long as a solvent that does not inhibit the reaction, but dissolves the starting materials is chosen, and examples include ethers such as diethyl ether, tetrahydrofuran and dioxane, amides such as dimethylformamide, dimethylacetamide and hexamethylphosphoric acid triamide, esters such as ethyl formate and ethyl acetate, sulfoxides such as dimethyl sulfoxide, and mixed solvents of these solvents. For the reaction of the acid halide and amino group of the peptide compound, an organic base such as triethylamine and pyridine can be added, if necessary.

The active esterification method is performed by reacting carboxyl group of the alcohol compound with an active esterifying agent in a solvent to prepare an active ester, and then reacting the active ester with amino group of the peptide compound. Examples of the solvent include halogenated hydrocarbons such as methylene chloride and chloroform, ethers such as diethyl ether and tetrahydrofuran, amides such as dimethylformamide and dimethylacetamide, aromatic hydrocarbons such as benzene, toluene and xylene, esters such as ethyl acetate, and mixed solvents of these solvent. Examples of the active esterifying agent include, for example, N-hydroxy compounds such as N-hydroxysuccinimide, 1-hydroxybenzotriazole and N-hydroxy-5-norbornene-2,3-dicarboxyimide; diimidazole compounds such as 1,1-oxalyldiimidazole and N,N'-carbonyldiimidazole; disulfide compounds such as 2,2'-dipyridyl disulfide; succinic acid compounds such as N,N'-disuccinimidyl carbonate; phosphinic chloride compounds such as N,N'-bis-(2-oxo-3-oxazolidinyl)phosphinic chloride; oxalate compounds such as N,N'-disuccinimidyl oxalate (DSO), N,N'-diphthalimide oxalate (DPO), N,N'-bis(norbornenylsuccinimidyl)oxalate (BNO), 1,1-bis(benzotriazolyl)oxalate (BBTO), 1,1-bis(6-chlorobenzotriazolyl)oxalate (BCTO) and 1,1-bis(6-trifluoromethylbenzotriazolyl)oxalate (BTBO), and the like.

The reaction of amino group of the peptide compound and the active ester is preferably performed in the presence of a condensing agent, for example, di(lower alkyl) azodicarboxylate/triphenylphosphines such as diethyl azodicarboxylate/triphenylphosphine, N-(lower alkyl)-5-aryl isoxazolium-3'-sulfonates such as N-ethyl-5-phenylisoxazolium-3'-sulfonate, oxydiformates such as diethyl oxydiformate (DEPC), N',N'-dicycloalkylcarbodiimides such as N',N'-dicyclohexylcarbodiimide (DCC), diheteroaryl diselenides such as di-2-pyridyl diselenide, triarylphosphines such as triphenylphosphine, arylsulfonyl triazolides such as p-nitrobenzenesulfonyl triazolide, 2-halo-1-(lower alkyl)pyridinium halide such as 2-chloro-1-methylpyridinium iodide, diarylphosphoryl azides such as diphenylphosphoryl azide (DPPA), imidazole derivatives such as N,N'-carbodiimidazole (CDI), benzotriazole derivatives such as 1-hydroxybenzotriazole (HOBT), dicarboxyimide derivatives such as N-hydroxy-5-norbornene-2,3-dicarboxyimide (HONB), carbodiimide derivatives such as 1-ethyl-3-(3-dimethylaminopropyl)carbodiimide (WSC), phosphonic acid cyclic anhydride such as 1-propanephosphonic acid cyclic anhydride (T3P), and the like. The reaction temperature for the preparation of the active ester is -10°C to room temperature, reaction temperature for the reaction of the active ester compound and amino group of the peptide compound is around room temperature, and the reaction time is 30 minutes to about 10 hours for both the reactions.

The mixed acid anhydride method is performed by preparing a mixed acid anhydride for carboxyl group of the alcohol compound, and then reacting the mixed acid anhydride with amino group of the peptide compound. The reaction for preparing the mixed acid anhydride can be performed in an inert solvent (for example, ethers such as diethyl ether and tetrahydrofuran, amides such as dimethylformamide and dimethylacetamide) by using a lower alkyl carbonate halide such as ethyl chlorocarbonate and isobutyl chlorocarbonate, a di(lower alkyl) cyanophosphate such as diethyl cyanophosphate, or the like. The reaction is preferably performed in the presence of an organic amine such as triethylamine and N-methylmorpholine, the reaction temperature is -10°C to room temperature, and the reaction time is about 30 minutes to 5 hours. The reaction of the mixed acid anhydride and amino group of the peptide compound is preferably performed in an inert solvent (for example, ethers such as diethyl ether and tetrahydrofuran, amides such as dimethylformamide and dimethylacetamide) in the presence of the aforementioned organic amine, the reaction temperature is 0°C to room temperature, and the reaction time is about 1 hour to 24 hours. Moreover, the condensation can also be attained by directly reacting a carboxylic acid and an amine compound in the presence of the aforementioned condensing agent. This reaction is performed in the same manner as that of the reaction for preparing active ester mentioned above.

Then, by reacting a reactive functional group (for example, carboxyl group, hydroxyl group, and the like) of the resulting peptide-bound alcohol compound and a reactive functional group (for example, carboxyl group, amino group, and the like) of a phospholipid compound, the phospholipid derivative of the present invention can be obtained. For example, when a phosphatidylethanolamine is used as the phospholipid compound, the phospholipid derivative of the present invention can be prepared by reacting amino group of the phospholipid compound and carboxyl group of the peptide end of the peptide-bound alcohol compound. This reaction can be performed in the same manner as that of the reaction explained above, and the reaction is preferably performed, for example, in the presence of a condensing agent according to the active ester method or the like.

As for the aforementioned reactions, an objective reaction may be efficiently performed by using a protective group. As for introduction of protective groups, for example, "Protective groups in organic synthesis", P.G.M. Wuts and T. Green, 3rd Edition, 1999, Wiley, John & Sons, and the like can be referred to. Isolation and purification of an objective compound can be performed by ordinary methods used in this field, and for example, purification by high performance liquid chromatography or the like is preferred. The phospholipid derivatives of the present invention include those in the form of a salt. Type of the salt is not particularly limited, and examples include mineral acid salts such as hydrochloride and sulfate, organic acid salts such as oxalate and acetate, metal salts such as sodium salt and potassium salt, ammonium salts, organic amine salts such as monomethylamine salt, and the like.

Type of the lipid membrane structure provided by the present invention is not particularly limited, and examples of the form in which lipid membrane structures are dispersed in an aqueous solvent include unilamella liposomes, multi-lamella liposomes, O/W type emulsions, W/O/W type emulsions, spherical micelles, fibrous micelles, layered structures of irregular shapes and the like. Among them, liposomes are preferred. The size of the lipid membrane structure in the dispersed state should not be particularly limited. For example, the particle diameter of liposomes or particles in emulsion is 50 nm to 5 $\mu$ m. The particle diameter of spherical micelle is 5 to 100 nm. Where a fibrous micelle or irregular layered structure is prepared, the thickness of one layer thereof is 5 to 10 nm, and such layers preferably form a single layer.

The lipid membrane structure provided by the present invention may further contain, in addition to the aforementioned phospholipid derivative provided by the present invention, ordinarily used phospholipids, a sterol such as cholesterol, and cholestanol, a fatty acid having a saturated or unsaturated acyl group having 8 to 22 carbon atoms and an antioxidant such as $\alpha$-tocopherol. Examples of the phospholipids include, for example, phosphatidylethanolamines, phosphatidylcholines, phosphatidylserines, phosphatidylinositols, phosphatidylglycerols, cardiolipins, sphingomyelins, ceramide phosphorylethanolamines, ceramide phosphorylglycerols, ceramide phosphorylglycerol phosphates, 1,2-dimyristoyl-1,2-deoxyphosphatidylcholines, plasmalogens, phosphatidic acids, and the like, and these may be used alone or two or more kind of them can be used in combination. The fatty acid residues of these phospholipids are not particularly limited, and examples thereof include a saturated or unsaturated fatty acid residue having 12 to 20 carbon atoms. Specific examples include an acyl group derived from a fatty acid such as lauric acid, myristic acid, palmitic acid, stearic acid, oleic acid and linoleic acid. Further, phospholipids derived from natural products such as egg yolk lecithin and soybean lecithin can also be used for the preparation of the lipid membrane structure of the present invention. Also usable are, for example, 1,2-bis(oleoyloxy)-3-(trimethylammonio)propane (DOTAP), 1-N,N-dimethylaminodioleoylpropane (DODAP), 1-oleoyl-2-hydroxy-3-N,N-dimethylaminopropane, 1,2-diacyl-3-N,N-dimethylaminopropane, 1,2-didecanoyl-1-N,N-dimethylaminopropane, 3-$\beta$-[n-[(N',N'-dimethylamino)ethane]carbamoyl]cholesterol (DC-Chol), 1,2-dimyristyloxypropyl-3-dimethylhydroxyethylammonium bromide (DMRIE), 1,2-dioleoyloxypropyl-3-dimethylhydroxyethylammonium bromide (DORI), and the like.

A lipid derivative having, for example, function for imparting retainability in blood, temperature change-sensitivity, pH-sensitivity or the like may be contained in the lipid membrane structure of the present invention as a membrane component lipid, one or more of these functions can be thereby imparted, and by imparting one or more of these functions, for example, blood retainability of the lipid membrane structure containing a medicament and/or a gene can be improved, a rate of capture by reticuloendothelial systems of liver, spleen and the like can be reduced, or a releasing property of the medicament and/or gene can be enhanced.

Examples of lipid derivatives retainable in blood, which can impart the function for imparting retainability in blood, include, for example, glycophorin, ganglioside GM1, phosphatidylinositol, ganglioside GM3, glucuronic acid derivative, glutamic acid derivative, polyglycerin-phospholipid derivative, polyethylene glycol derivatives such as N-{carbonyl-methoxypolyethylene glycol-2000}-1,2-dipalmitoyl-sn-glycero-3-phosphoethanolamine, N-{carbonyl-methoxypolyethylene glycol-5000}-1,2-dipalmitoyl-sn-glycero-3-phosphoethanolamine, N-{carbonyl-methoxypolyethylene glycol-750}-1,2-distearoyl-sn-glycero-3-phosphoethanolamine, N-{carbonyl-methoxypolyethylene glycol-2000}-1,2-distearoyl-sn-glycero-3-phosphoethanolamine and N-{carbonyl-methoxypolyethylene glycol-5000}-1,2-distearoyl-sn-glycero-3-phosphoethanolamine, and the like.

Examples of temperature change-sensitive lipid derivatives that can impart the temperature change-sensitive function include, for example, dipalmitoylphosphatidylcholine and the like. Examples of pH-sensitive lipid derivatives that can impart the pH-sensitive function include, for example, dioleoylphosphatidylethanolamine and the like.

Although the form of the lipid membrane structure of the present invention is not particularly limited, for example, a form in which the phospholipid derivative of the present invention forms the lipid membrane structure together with the phospholipid as a membrane component of the lipid membrane structure is preferred. More specifically, examples include, for example, a form in which the phospholipid derivative of the present invention exists (binds) at one or more kinds of positions selected from the group consisting of positions in the lipid membrane, on the lipid membrane surface of the lipid membrane structure, in an internal space of the lipid membrane structure, in a lipid layer, and on a lipid layer surface in a lipid membrane structure constituted by other phospholipids. More preferred examples include a form in which the phospholipid derivative of the present invention serves as a membrane component together with other phospholipid and the like to form the lipid membrane structure such as liposome.

The form and production method of the lipid membrane structure of the present invention are not particularly limited. Examples of the form include a dry mixture form, a form in which the lipid membrane structure is dispersed in an aqueous solvent, a form obtained by drying or freezing any of the forms mentioned above and the like. The methods for producing the lipid membrane structures of these forms will be explained below. However, the form of the lipid membrane structure of the present invention and the methods for preparing thereof are not limited to the aforementioned forms and the production methods explained below.

Although the method for preparing the lipid membrane structure of the present invention is not particularly limited, for example, the lipid membrane structure in the form of dried mixture can be produced by, for example, once dissolving all the components of the lipid membrane structure in an organic solvent such as chloroform and then subjecting the resulting solution to solidification under reduced pressure by using an evaporator or spray drying by using a spray dryer. The form of the lipid membrane structure dispersed in an aqueous solvent can be prepared by adding the aforementioned dried mixture to an aqueous solvent and emulsifying the mixture by using an emulsifier such as a homogenizer, ultrasonic emulsifier, high pressure jet emulsifier or the like. Further, the aforementioned form can also be prepared by a method known as a method for preparing liposomes, for example, the reverse phase evaporation method or the like. When it is desired to control a size of the lipid membrane structure, extrusion (extrusion filtration) can be performed under high pressure by using a membrane filter of uniform pore sizes or the like.

The composition of the aqueous solvent (dispersion medium) should not be particularly limited, and examples include, for example, a buffer such as phosphate buffer, citrate buffer, and phosphate-buffered physiological saline, physiological saline, a medium for cell culture and the like. Although the lipid membrane structure can be stably dispersed in these aqueous solvents (dispersion media), the solvents may be further added with a saccharide (aqueous solution), for example, a monosaccharide such as glucose, galactose, mannose, fructose, inositol, ribose and xylose, disaccharide such as lactose, sucrose, cellobiose, trehalose and maltose, trisaccharide such as raffinose and melezitose, and polysaccharide such as cyclodextrin, sugar alcohol such as erythritol, xylitol, sorbitol, mannitol, and maltitol, or a polyhydric alcohol (aqueous solution) such as glycerin, diglycerin, polyglycerin, propylene glycol, polypropylene glycol, ethylene glycol, diethylene glycol, triethylene glycol, polyethylene glycol, ethylene glycol mono-alkyl ether, diethylene glycol mono-alkyl ether and 1,3-butylene grycol. In order to stably store the lipid membrane structure dispersed in such an aqueous solvent (dispersion medium) for a long period of time, it is desirable to minimize electrolytes in the aqueous solvent (dispersion medium) from a viewpoint of physical stability such as prevention of aggregation. Further, from a viewpoint of chemical stability of lipids, it is desirable to control pH of the aqueous solvent (dispersion medium) to be in a range of from weakly acidic pH to around neutral pH (pH 3.0 to 8.0), and to remove dissolved oxygen by nitrogen bubbling.

Further, the dried or frozen form of the form in which the lipid membrane structure is dispersed in an aqueous solvent can be produced by drying or freezing the aforementioned lipid membrane structure dispersed in an aqueous solvent by an ordinary drying or freezing method based on lyophilization or spray drying. When a lipid membrane structure dispersed in the aqueous solvent is first prepared and then successively dried, it becomes possible to store the lipid membrane structure for a long period of time. In addition, when an aqueous solution containing a medicinally active ingredient is added to the dried lipid membrane structure, the lipid mixture is efficiently hydrated and thereby the medicinally active ingredient can be efficiently retained in the lipid membrane structure, which provides an advantageous effect.

When lyophilization or spray drying is carried out, a use of a saccharide (as an aqueous solution), for example, a monosaccharide such as glucose, galactose, mannose, fructose, inositol, ribose and xylose, disaccharide such as lactose, sucrose, cellobiose, trehalose and maltose, trisaccharide such as raffinose and melezitose, and polysaccharide such as cyclodextrin, or a sugar alcohol such as erythritol, xylitol, sorbitol, mannitol, and maltitol may achieve stable storage of the lipid membrane structure for a long period of time. For the freezing, a use of the aforementioned saccharide (as an aqueous solution) or a polyhydric alcohol (aqueous solution) such as glycerin, diglycerin, polyglycerin, propylene glycol, polypropylene glycol, ethylene glycol, diethylene glycol, triethylene glycol, polyethylene glycol, ethylene glycol mono-alkyl ether, diethylene glycol mono-alkyl ether and 1,3-butylene glycol may achieve stable storage of the lipid membrane structure for a long period of time. A saccharide and a polyhydric alcohol may be used in combination. A concentration of the saccharide or polyhydric alcohol in the form in which the lipid membrane structure is dispersed in an aqueous solvent is not particularly limited. In a state that the lipid membrane structure is dispersed in an aqueous solvent, for example, the concentration of the saccharide (aqueous solution) is preferably 2 to 20% (W/V), more preferably 5 to 10% (W/V), and the concentration of the polyhydric alcohol (aqueous solution) is preferably 1 to 5% (W/V), more preferably 2 to 2.5% (W/V). When a buffer is used as the aqueous solvent (dispersion medium), a concentration of the buffering agent is preferably 5 to 50 mM, more preferably 10 to 20 mM. The concentration of the lipid membrane structure in an aqueous solvent (dispersion medium) should not be particularly limited. However, the concentration of the total amount of lipids in the lipid membrane structure is preferably 0.1 to 500 mM, more preferably 1 to 100 mM.

In the lipid membrane structure of the present invention, a medicament such as an antitumor agent and/or a gene for gene therapy of malignant tumor and the like can be retained. The term "retain" used herein means that the antitumor agent and/or gene is present in a lipid membrane, on a surface of lipid membrane, in an internal space of lipid membrane, in a lipid layer and/or on a surface of lipid layer of the lipid membrane structure. When the lipid membrane structure is a microparticle such as liposome, the antitumor agent and/or the gene can also be encapsulated in the inside of the microparticle. The amount of the antitumor agent and/or gene to be retained in the microparticle is not particularly limited, and the amount may be that sufficient for effectively expressing pharmacological activity thereof in an organism (or in cells). The type of the antitumor agent and/or the gene is not also particularly limited, and may be suitably determined depending on type of malignant tumor, form of the lipid membrane structure, and the like.

Examples of the antitumor agent include, for example, camptothecin derivatives such as irinotecan hydrochloride, nogitecan hydrochloride, exatecan, RFS-2000, lurtotecan, BNP-1350, Bay-383441, PNU-166148, IDEC-132, BN-80915, DB-38, DB-81, DB-90, DB-91, CKD-620, T-0128, ST-1480, ST-1481, DRF-1042 and DE-310, taxane derivatives such as docetaxel hydrate, paclitaxel, IND-5109, BMS-184476, BMS-188797, T-3782, TAX-1011, SB-RA-31012, SBT-1514 and DJ-927, ifosfamide, nimustine hydrochloride, carboquone, cyclophosphamide, dacarbazine, thiotepa, busulfan, melphalan, ranimustine, estramustine phosphate sodium, 6-mercaptopurine riboside, enocitabine, gemcitabine hydrochloride, carmofur, cytarabine, cytarabine ocphosphate, tegafur, doxifluridine, hydroxycarbamide, fluorouracil, methotrexate, mercaptopurine, fludarabine phosphate, actinomycin D, aclarubicin hydrochloride, idarubicin hydrochloride, epirubicin hydrochloride, daunorubicin hydrochloride, doxorubicin hydrochloride, pirarubicin hydrochloride, bleomycin hydrochloride, zinostatin stimalamer, neocarzinostatin, mytomycin C, bleomycin sulfate, peplomycin sulfate, etoposide, vinorelbine tartrate, vincristine sulfate, vindesine sulfate, vinblastine sulfate, amrubicin hydrochloride, gefitinib, exemestan, capecitabine, TNP-470, TAK-165, KW-2401, KW-2170, KW-2871, KT-5555, KT-8391, TZT-1027, S-3304, CS-682, YM-511, YM-598, TAT-59, TAS-101, TAS-102, TA-106, FK-228, FK-317, E7070, E7389, KRN-700, KRN-5500, J-107088, HMN-214, SM-11355, ZD-0473 and the like.

The gene means a nucleic acid, and may be any of oligonucleotide, DNA, and RNA, and examples thereof include a gene that exhibits anti-malignant tumor action upon in vivo expression, for example, a gene for gene therapy of malignant tumor, and the like. Examples of the gene for gene therapy include an antisense oligonucleotide, antisense DNA, antisense RNA, shRNA, and siRNA involved in angiogenesis or cell proliferation in malignant tumor, a gene coding for a physiologically active substance such as enzymes and cytokines, antisense RNA, shRNA, or siRNA, and the like.

When the lipid membrane structure contains a gene, it is preferable to add a compound having a gene transfer function as a component of the lipid membrane structure to efficiently introduce the gene into a cell. Examples of such a compound include O,O'-N-didodecanoyl-N-($\alpha$-trimethylammonioacetyl)-diethanolamine chloride, O,O'-N-ditetradecanoyl-N-($\alpha$-trimethylammonioacetyl)-diethanolamine chloride, O,O'-N-dihexadecanoyl-N-($\alpha$-trimethylammonioacetyl)-diethanolamine chloride, O,O'-N-dioctadecenoyl-N-($\alpha$-trimethylammonioacetyl)-diethanolamine chloride, O,O',O"-tridecanoyl-N-($\omega$-trimethylammoniodecanoyl)aminomethane bromide, N-[$\alpha$-trimethylammonioacetyl]-didodecyl-D-glutamate, dimethyldioctadecylammonium bromide, 2,3-dioleoyloxy-N-[2-(sperminecarboxamido)ethyl]-N,N-dimethyl-1-propaneammonium trifluoroacetate, 1,2-dimyristyloxypropyl-3-dimethylhydroxyethylammonium bromide, 3-$\beta$-[N-(N', N'-dimethylaminoethane)carbamoyl]cholesterol, and the like. A form is preferred in which any of the compounds having the gene transfer function is present (binds) in a membrane, on a surface of membrane, in a internal space of membrane, in a lipid layer and/or on a surface of lipid layer of the lipid membrane structure.

Moreover, the lipid membrane structure may contain an antibody that specifically recognizes a malignant tumor cell or matrix metalloproteinase. As the antibody, a monoclonal antibody is preferred. For example, one kind of monoclonal antibody directed to a single epitope may be used, or a combination of two or more kinds of monoclonal antibodies having specificity for various epitopes may also be used. Moreover, the antibody may be a monovalent antibody or a multivalent antibody, and an naturally occurring type (intact) molecule, or a fragment or derivative thereof may be used. For example, a fragment such as F(ab')$_2$, Fab' and Fab may be used, and a chimeric antibody or hybrid antibody having at least two of antigen- or epitope-binding sites, a double specificity recombinant antibody such as quadrome and triome, an interspecies hybrid antibody, an anti-idiotype antibody and a chemically modified or processed version of these considered as a derivative of any of the foregoing antibodies may also be used. Further, those that may be used include, for example, an antibody obtained by a synthetic or semisynthetic technique with applying a known cell fusion or hybridoma technique or a known antibody engineering technique, an antibody prepared by using a DNA recombinant technique by applying a conventional technique known from a viewpoint of antibody production, and an antibody having a neutralization or binding property for a target epitope.

The lipid membrane structure of the present invention retaining an antitumor agent and/or a gene can be used as a pharmaceutical composition for therapeutic treatment of a malignant tumor. The existing form of the pharmaceutical composition of the present invention and methods for preparation thereof are not particularly limited, and the composition may be produced in the same form as the aforementioned lipid membrane structure. For example, examples of the form include a dried mixture form, a form of dispersion in an aqueous solvent, and a form obtained by drying or freezing the previously mentioned form.

The form of dried mixture can be produced by once dissolving the components of the lipid membrane structure and an antitumor agent and/or a gene in an organic solvent such as chloroform to obtain a mixture, and then subjecting the mixture to solidification under reduced pressure by using an evaporator or spray drying by using a spray dryer. Several methods are known as methods for producing a mixture of lipid membrane structures and a medicinally active ingredient such as an antitumor agent and/or a gene in the form of dispersion in an aqueous solvent. It is possible to appropriately chose a suitable method depending on the mode of retaining an antitumor agent and/or a gene, properties of the mixture and the like of the lipid membrane structure as follows.

Production Method 1

Production Method 1 is a method of adding an aqueous solvent to the aforementioned dried mixture and emulsifying the mixture by using an emulsifier such as homogenizer, ultrasonic emulsifier, high-pressure injection emulsifier, or the like. When it is desired to control the size (particle diameter), extrusion (extrusion filtration) can be further performed under a high pressure by using a membrane filter having uniform pore sizes. In this method, in order to prepare a dried mixture of components of the lipid membrane structure and an antitumor agent and/or a gene first, it is necessary to dissolve the lipid membrane structure and the antitumor agent and/or gene in an organic solvent, and the method has an advantage that it can make the best utilization of interactions between the antitumor agent and/or the gene and components of the lipid membrane structure. More specifically, even when the lipid membrane structures have a layered structure, the antitumor agent and/or gene can enter into the inside of the multiple layers, and thus use of this method generally provides a higher retention ratio of the antitumor agent and/or gene in the lipid membrane structures.

Production Method 2

Production Method 2 is a method of adding an aqueous solvent containing an antitumor agent and/or a gene to dried components of the lipid membrane structure obtained by dissolving the components in an organic solvent and evaporating the organic solvent, and emulsifying the mixture to attain the production. When it is desired to control the size (particle diameter), extrusion (extrusion filtration) can be further performed under a high pressure by using a membrane filter having uniform pore sizes. This method can be used for an antitumor agent and/or a gene that is hardly dissolved in an organic solvent, but can be dissolved in an aqueous solvent. When the lipid membrane structures are liposomes, they have an advantage that they can retain an antitumor agent and/or a gene also in the part of internal aqueous phase.

Production Method 3

Production Method 3 is a method of further adding an aqueous solvent containing an antitumor agent and/or a gene to lipid membrane structures such as liposomes, emulsions, micelles or layered structures already dispersed in an aqueous solvent. This method is limitedly applied to a water-soluble antitumor agent and/or gene. In this method, the addition of an antitumor agent and/or a gene to already prepared lipid membrane structures is performed from the outside. Therefore, when the antitumor agent and/or gene is a polymer, the antitumor agent and/or gene may not enter into the inside of the lipid membrane structures, and the an antitumor agent and/or gene may be present in a form that it is present on (binds to) the surfaces of lipid membrane structures. It is known that when liposomes are used as the lipid membrane structures, use of Production Method 3 may result in formation of a sandwich-like structure in which the antitumor agent and/or gene is sandwiched between liposome particles (generally called as a complex). An aqueous dispersion of lipid membrane structures alone is prepared beforehand in this production method. Therefore, decomposition of an antitumor agent and/or a gene during the emulsification need not be taken into consideration, and a control of the size (particle diameter) is also readily operated, which enables relatively easier preparation compared with Production Methods 1 and 2.

Production Method 4

Production Method 4 is a method of further adding an aqueous solvent containing an antitumor agent and/or a gene to a dried product obtained by once producing lipid membrane structures dispersed in an aqueous solvent and then drying the same. In this method, the antitumor agent and/or gene is limited to a water-soluble antitumor agent and/or a gene as in Production Method 3. A significant difference from Production Method 3 is the modes of presence of the lipid membrane structures and the antitumor agent and/or gene. That is, in Production Method 4, lipid membrane structures dispersed in an aqueous solvent are once produced and further dried to obtain a dried product, and at this stage, the lipid membrane structures are present in a state of a solid as fragments of lipid membranes. In order to allow the fragments of lipid membranes to be present in a solid state, it is preferable to use a solvent added with a sugar (aqueous solution), preferably sucrose (aqueous solution) or lactose (aqueous solution), as the aqueous solvent as described above. In this method, when the aqueous solvent containing an antitumor agent and/or a gene is added, hydration of the fragments of the lipid membranes present in a state of a solid quickly starts with the invasion of water, and thus the lipid membrane structures can be reconstructed. At this time, a structure of a form in which an antitumor agent and/or a gene is retained in the inside of the lipid membrane structures can be produced.

In Production Method 3, when the antitumor agent and/or gene is a polymer, the antitumor agent and/or gene cannot enter into the inside of the lipid membrane structures, and is present in a mode that it binds to the surfaces of the lipid membrane structures. Production Method 4 significantly differs in this point. In Production Method 4, an aqueous dispersion of lipid membrane structures alone is prepared beforehand, and therefore, decomposition of the antitumor agent and/or gene during the emulsification need not be taken into consideration, and a control of the size (particle diameter) is also easily attainable. For this reason, said method enables relatively easier preparation compared with Production Methods 1 and 2. Besides the above mentioned advantages, this method also has advantages that storage stability for a pharmaceutical preparation (or pharmaceutical composition) is easily secured, because the method uses lyophilization or spray drying; when the dried preparation is rehydrated with an aqueous solution of an antitumor agent and/or a gene, original size (particle diameter) can be reproduced; even when a polymer antitumor agent and/or gene is used, the antitumor agent and/or gene can be easily retained in the inside of the lipid membrane structures and the like.

As other method for producing a mixture of lipid membrane structures and an antitumor agent and/or a gene in a form of a dispersion in an aqueous solvent, a method well known as that for producing liposomes, e.g., the reverse phase evaporation method or the like, may be separately used. When it is desired to control the size (particle diameter), extrusion (extrusion filtration) can be performed under a high pressure by using a membrane filter having uniform pore sizes. Further, examples of the method for further drying a dispersion, in which the aforementioned mixture of lipid membrane structures and an antitumor agent and/or a gene is dispersed in an aqueous solvent, include lyophilization and spray drying. As the aqueous solvent in this process, it is preferable to use the aforementioned solvent added with a sugar (as an aqueous solution), preferably sucrose (as an aqueous solution) or lactose (as an aqueous solution). Examples of the method for further freezing a dispersion, in which the aforementioned mixture of lipid membrane structures and an antitumor agent and/or a gene is dispersed in an aqueous solvent, include ordinary freezing methods. As the aqueous solvent in this process, it is preferable to use a solvent added with a sugar (as an aqueous solution) or polyhydric alcohol (aqueous solution).

Production Method 5

As for lipid membrane structures in which antibodies are retained on surfaces of the lipid membrane structures, by producing lipid membrane structures using components of the lipid membrane structures and an antitumor agent and/or a gene and then adding an antibody in a manner similar to any of those of Production Methods 1 to 4, a composition in a form where the antibody is present on (or binds to) the surfaces of the membranes of lipid membrane structures can be produced.

Production Method 6

As for lipid membrane structures in which antibodies are retained on surfaces of the lipid membrane structures, by producing lipid membrane structures using components of the lipid membrane structures and an antitumor agent and/or a gene and then adding an antibody and a lipid derivative that can react with mercapto group in the antibody in a manner similar to any of those of Production Methods 1 to 4, a composition in a form where the antibody is present on (or binds to) the surfaces of the membranes of lipid membrane structures can be produced.

Lipids which can be added in the preparation of the lipid membrane structure of the present invention may be suitably chosen depending on a type of the antitumor agent and/or gene and the like to be used. For example, when an antitumor agent is used, the lipids are used in an amount of, for example, 0.1 to 1000 parts by mass, preferably 0.5 to 200 parts by mass, in terms of the total lipid amount, on the basis of 1 part by mass of the antitumor agent. When a gene is used, the amount is preferably 1 to 500 nmol, more preferably 10 to 200 nmol, in terms of the total lipid amount, on the basis of 1 $\mu$g of the gene.

The pharmaceutical composition of the present invention containing the lipid membrane structure retaining an antitumor agent and/or a gene for gene therapy of a malignant tumor is useful for therapeutic treatment of a tumor. Although type of malignant tumor curable with the pharmaceutical composition of the present invention is not particularly limited, malignant tumors expressing particularly much matrix metalloproteinase are suitable. Examples of malignant tumor cell include cells of fibrosarcoma, squamous carcinoma, neuroblastoma, breast carcinoma, gastric cancer, hepatoma, bladder cancer, thyroid tumor, urinary tract epithelial cancer, glioblastoma, acute myeloid leukemia, pancreatic duct cancer, prostate cancer and the like, but not limited to these cells. When the pharmaceutical composition is administered to an animal such as a human or experimental cells, an antitumor agent and/or a gene can be efficiently delivered to an angiogenesis front inside a tumor. Examples of the angiogenesis front inside a tumor include endothelial cells of ruffling edge and the like, but not limited to these examples.

Although it is not intended to be bound by any specific theory, when the pharmaceutical composition of the present invention is administered to a mammal including human, superior blood retainability of the lipid membrane structure is achieved due to the presence of the modification moiety such as poly(alkylene glycol), whilst in the vicinity of malignant tumor cells secreting a matrix metalloproteinase, the modification moiety is dissociated and thereby the antitumor agent contained in the pharmaceutical composition of the present invention is released, and the pharmaceutical composition becomes more likely to be taken up by the malignant tumor cells. Accordingly, the effect of the antitumor agent and/or gene can be exerted on the cells.

The administration method of the pharmaceutical composition containing the lipid membrane structures of the present invention is not particularly limited, and either oral administration or parenteral administration may be used. Examples of dosage forms for oral administration include, for example, tablets, powders, granules, syrups, capsules, solutions for internal use and the like, and examples of dosage forms for parenteral administration include, for example, injections, drip infusion, eye drops, ointments, suppositories, suspensions, cataplasms, lotions, aerosols, plasters and the like. Injection or drip infusion is preferred among them, and administration methods include intravenous injection, arterial injection, subcutaneous injection, intradermal injection and the like, as well as local injection to targeted cells or organs. Doses, administration period and the like of the pharmaceutical composition of the present invention are not particularly limited, and suitable dose and administration period can be chosen depending on various conditions including type and retaining amount of the antitumor agent and/or gene acting as an active ingredient, type of the lipid membrane structure, type of malignant tumor, patient's body weight, age, and the like.

Examples

The present invention will be explained more specifically with reference to the following examples. However, the scope of the present invention is not limited to these examples.

Example 1

NHS was purchased from Wako Pure Chemical Industries. DCC was purchased from Kanto Kagaku. For GPC, used were Tosoh 8120 (TOSOH CORP.), Tosoh HLC-8120 (RI) as a detector, TSK-Gel Super HZ3000 and 2500 as columns, and THF as a carrier. The flow rate was 0.35 mL/minute, and the temperature was 40°C. $^1$H-NMR measurement was performed by using JEOL EX-400 (400 MHz). TOF-MS measurement was performed by using Bruker MALDI-TOF-MS Reflex II. A dialysis membrane, Spectra/Por Membren MWCO:1,000, was purchased from Spectrum. A peptide was purchased from the Greiner Japan. The sequence thereof was Gly-Gly-Gly-Val-Pro-Leu-Ser-Leu-Tyr-Ser-Gly-Gly-Gly-Gly, and a molecular weight thereof was 1178.9.

(1) Synthesis of polyethylene glycol (PEG)

In an argon atmosphere, tetrahydrofuran (THF, 20 mL) was put into a recovery flask, and 80 μL of 2-methoxyethanol was added as a polymerization initiator with stirring by a stirrer. Then, 2.9 mL of potassium naphthalenide (0.342 mol/L) was added. Subsequently, 2.3 mL (45 mmol) of ethylene oxide (EO) was added. The reaction was allowed at ordinary temperature under ordinary pressure for 48 hours with stirring. After the reaction for 48 hours, a part of the reaction solution (about 2 mL) was collected, and the molecular weight of the product was measured by gel permeation chromatography (GPC). The number average molecular weight (Mn) was 1,678, and the weight average molecular weight (Mw) was 1,825. Further, the polydispersity index calculated as Mw/Mn was as small as 1.078, and a monodispersed peak was observed in GPC, and therefore, it was judged that polymerization of PEG sufficiently advanced.

(2) Carboxylation of PEG end

A half volume (12.5 mL) of the reaction mixture obtained in (1) mentioned above was put into a recovery flask in an argon atmosphere. To the reaction mixture was added 3.5 mL of succinic anhydride (0.727 mol/L), and the mixture was stirred by a stirrer to perform a PEG polymerization termination reaction at ordinary temperature for 24 hours. After the reaction for 24 hours, the reaction product was precipitated by adding diethyl ether (1 L) to the reaction mixture. The precipitates were collected by filtration, taken into a beaker, and dissolved with methanol. The product was precipitated again with diethyl ether (1 L), the precipitates were collected by filtration, and dissolved with methanol, and the solution was dialyzed against distilled water. After the dialysis, the solution was transferred to a recovery flask, and lyophilized to obtain pale yellow powder (0.904 g). The molecular weight was measured by GPC to find that Mn was 1,773, and Mw was 1,903. The polydispersity index of the product was 1.073, and a monodispersed peak was obtained. Accordingly, it was confirmed that the reaction advanced without side reaction. When $^1$H-NMR spectrum was measured, the integral ratio of the peak originated in methoxy group (OMe) around 3.4 ppm and the peak originated in -CO-$CH_2CH_2$-CO- around 2.6 ppm was about 1:1, and thus it was confirmed that carboxylic acid had been introduced at the end.

$^1$H-NMR ($CDCl_3$) δ (ppm) 2.62 (s, CO$CH_2CH_2$CO, 4H), 3.38 (s, 3H), 3.65 (s, 176H), 4.26 (t, 2H).

(3) Active esterification of carboxyl group at PEG end (synthesis of MeO-PEG-NHS)

The compound obtained in (2) mentioned above in an amount of 0.2 g was put into a 50-mL screw tube. To the compound in the screw tube was added 57.6 mg of NHS, 41.26 mg of DCC, and 10 mL of chloroform, and the reaction was allowed at ordinary temperature for 24 hours with stirring by a stirrer. After completion of the reaction, the reaction mixture was filtered to remove solid, and the reaction product was precipitated from the filtrate with 300 mL of diethyl ether. The precipitates were collected by filtration, and dried, and the resulting solid was taken into a beaker, and dissolved in chloroform. Precipitation with diethyl ether and collection by filtration were repeated twice, the obtained solid was dissolved in chloroform, and the solution was put into a 100-mL recovery flask. After the solvent was evaporated, benzene was added to the residue, and the mixture was lyophilized to obtain pale brown powder (104 mg). Mn measured by TOF-MS was 2192.12, and the polydispersity index was 1.02.

From the TOF-MS spectrum, it was confirmed that each interval of the peaks was 44, which corresponded to the molecular weight of each unit of PEG. By comparing the measured values observed in the TOF-MS spectrum and theoretical values of the peaks, it can be determined whether the objective functional group was introduced at the end. The theoretical values and measured values of the molecular weight for the various numbers of n are shown in Table 1. The measurement error for each n was 2 mass or less, indicating that the objective compound was obtained. Moreover, when $^1$H-NMR spectrum was measured, the introduction rate of the NHS group (integral ratio of the peak originating in the methoxy group at 3.37 ppm and the peak originating in the NHS group at 2.84 ppm) was 97.3%, and thus was successfully confirmed that the NHS group was introduced at a rate of substantially 100%.

$^1$H-NMR ($CDCl_3$) δ (ppm) 2.78 (t, 2H), 2.84 (s, 4H), 2.95 (t, 2H), 3.37 (s, 3H), 3.64 (s, 176H), 4.26 (t, 2H)

[Table 1]

| Value of n | Calcd. | Found | Error |
|---|---|---|---|
| 36 | 1926.16 | 1927.35 | 1.19 |
| 37 | 1970.21 | 1971.73 | 1.52 |
| 38 | 2014.27 | 2015.71 | 1.44 |
| 39 | 2058.32 | 2060.20 | 1.88 |
| 40 | 2102.37 | 2103.94 | 1.57 |

(continued)

| Value of n | Calcd. | Found | Error |
|---|---|---|---|
| 41 | 2146.42 | 2148.02 | 1.60 |

(4) Synthesis of PEG-peptide

To 24.6 mg of the peptide, 1 mL of dimethyl sulfoxide (DMSO), 41.17 mg of the compound obtained in (3) mentioned above, and 5 $\mu$ L of triethylamine were added, and the mixture was reacted at ordinary temperature for 3 hours with shaking. Completion of the reaction was confirmed by HPLC, and the reaction mixture was lyophilized to obtain white powder (66.76 mg). Although Mn of the product measured by TOF-MS was 3223.82, which was different by 32 mass from the theoretical value of Mn, i.e., 3255.93, it was estimated that the compound was derived from PEG and consisted of a PEG-peptide, because the compound had the molecular weight distribution. The polydispersity index was found to be 1.01, and a monodispersed peak was observed, and accordingly, it was confirmed that the reaction quantitatively advanced. When $^1$H-NMR spectrum was measured, a peak originating in the benzene ring of tyrosine in the peptide was confirmed at 6.6 to 7.4 ppm. The molar integral ratio of the peak originating in the methoxy group around 3.38 ppm and the peak originating in the benzene ring of tyrosine was 1:1, and thus it was successfully confirmed that the peptide was introduced to the PEG end at a ratio of 100%.

(5) Synthesis of PEG-peptide-DOPE

The PEG-peptide obtained in (4) of this example in an amount of 54.7 mg was dissolved in 4 mL of chloroform, 24.7 mg of DOPE, 6.85 mg of DCC, 3.82 mg of NHS and 4.63$\mu$ L of triethylamine were added to the solution, and the reaction was performed at ordinary temperature for 48 hours with shaking. After completion of the reaction, chloroform was removed by using a desiccator, and the residue was dissolved in 1.5 mL of chloroform again. The chloroform solution was slowly added dropwise to 100 mL of diethyl ether with stirring by a stirrer to precipitate the compound originating in PEG. The mixture was left standing at ordinary temperature until solid was precipitated, and the supernatant was removed. To the precipitates was added 50 mL of diethyl ether, the mixture was stirred by using a stirrer and left until solid was precipitated, and the supernatant was removed. The precipitates were dissolved in water, separated and purified by HPLC, and the collected fraction was dialyzed and lyophilized. Mn obtained by TOF-MS was 3955.15, and the polydispersity index was 1.00. The compound was found to be derived from PEG because the peak had the molecular weight distribution, and consisted of PEG-peptide-DOPE because the deviation of the measured Mn from the theoretical value, 3949.86, was as small as 5.3 mass. When $^1$H-NMR spectrum was measured, the integral ratio of the peak originating from the methoxy group at the end of PEG at 3.35 ppm and the peak originating from the benzene ring of tyrosine in the peptide at 6.6 to 7.1 ppm was 3:4.3, or 1:1 in terms of molar ratio, which values were not significantly different from those observed for the PEG-peptide.

Example 2

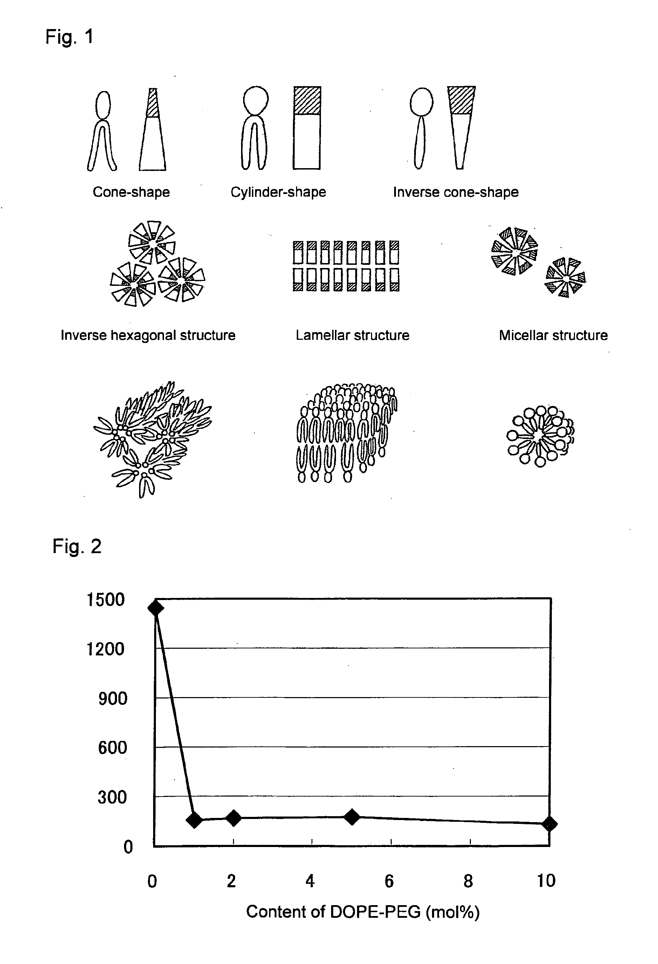

It was evaluated whether or not the PEG-peptide-DOPE obtained in Example 1 was cleavable with MMP. For in vitro evaluation, a system was used with which the cleavage of PEG was detected on the basis of an increase in particle diameter. The DOPE molecule has a cone-shaped structure with a hydrophobic group larger than a hydrophilic group, and DOPE molecules alone do not form liposomes, but take micelle-like inverse hexagonal structures in which hydrophobic groups face outward, and in an aqueous phase, the micelles aggregate and are thereby stabilized. Cylinder-shaped lipids form a lamellar structure (bilayer structure), i.e., a liposome structure, whereas inverse cone-shape lipids form a micellar structure (Fig. 1). It is known that when a PEG-lipid is added at a certain ratio to DOPE, the lipids form a lamellar structure and stably form liposomes. Therefore, if a matrix metalloproteinase is made to act on DOPE-containing liposomes prepared by using PEG-peptide-DOPE, thereby the peptide is decomposed and PEG is cleaved, DOPE molecules from which PEG is cleaved can no longer form liposomes, and take the hexagonal structure to initiate the aggregation. The above hypothesis was adopted as an evaluation method for PEG cleavage. Further, for in vitro evaluation of PEG cleavage, a method of measuring genetic expression activity was adopted. If the interaction of MEND and cell membranes is enhanced by cleavage of PEG, reduction of genetic expression activity is suppressed.

Matrix metalloproteinase 2 (MMP-2) was purchased from Sigma. DOPE-PEG was purchased from Avanti. Trypsin was purchased from Life Technologies. DISMIC was purchased from Advantec. Reverse phase HPLC was performed by using Model 1321H1 (GILSON), UV/VIS-155 (GILSON) as a detector, SOSMOSIL 5C$_{18}$-AR-II (Nacalai Tesque) as a column, and 0.5% TFA-H$_2$O and 0.5% TFA-acetonitrile filtered through Millicup-LH vacuum-driven bottletop filter unit (Millipore) as a mobile phase. The flow rate was 1.0 mL/minute.

(1) Preparation of DOPE liposomes and evaluation of PEG cleavage

A 1 mM lipid solution dissolved in ethanol, of which 125 $\mu$ L was taken as 100%, was put into test tubes at various molar ratios, and a 1 mM DOPE-PEG solution was also added as required. Chloroform was added in the same volume of ethanol to the mixture in each tube, and the mixture was stirred. Then, the solvent was evaporated in a desiccator to obtain a lipid membrane. After the preparation of the lipid membrane, a solution (250 $\mu$ L) containing 20 mM HEPES (pH 7.4), 280 mM NaCl, and 4 mM $CaCl_2$ was added, and after hydration for 30 minutes, the mixture was sonicated for 1 minute to prepare liposomes.

First, it was examined whether or not the peptide was decomposed by MMP-2. A 0.5 mM peptide solution (25 $\mu$ L) dissolved in a solution containing 10 mM HEPES (pH 7.4), 140 mM NaCl and 2 mM $CaCl_2$ was analyzed by reverse phase HPLC. Further, MMP2 was added to the 0.5 mM peptide solution (25 $\mu$ L) at a final concentration of 53 nM, 230 nM and 450 nM, each mixture was incubated at 37°C for 2 hours, and then the whole volume of the mixture was analyzed by HPLC to confirm whether or not the peptide was decomposed by MMP2. After the MMP-2 treatment, a peak was observed at a time earlier than that of the peak of the peptide. The observed peak became larger in an enzyme concentration-dependent manner, and thus it was demonstrated that the peak was derived from a degradation product provided by the enzyme.

Then, to the prepared liposomes (50 $\mu$ L), PBS (10 $\mu$ L), 230 nM or 5.5 nM MMP-2 in terms of final concentration, and 230 nM bovine serum albumin in terms of final concentration were added, the mixture was incubated at 37°C, and after 2, 8 and 24 hours, the particle diameter was measured. The relationship between the addition ratio of DOPE-PEG and the particle diameter is shown in Fig. 2. With 0% of DOPE-PEG, aggregates having a particle diameter exceeding 1,000 nm were observed, suggesting that these aggregates formed inverse hexagonal structure. Even with the addition of 1% of DOPE-PEG, liposomes having a particle size less than 200 nm and showing stable dispersion was successfully prepared. Further, the particle diameter gradually decreased in a PEG addition amount-dependent manner. Even after incubation of these liposomes at 37°C for 24 hours, aggregation was not observed. These results suggest that the phase transition temperature was elevated to a temperature higher than 37°C by the addition of 0.5% DOPE-PEG.

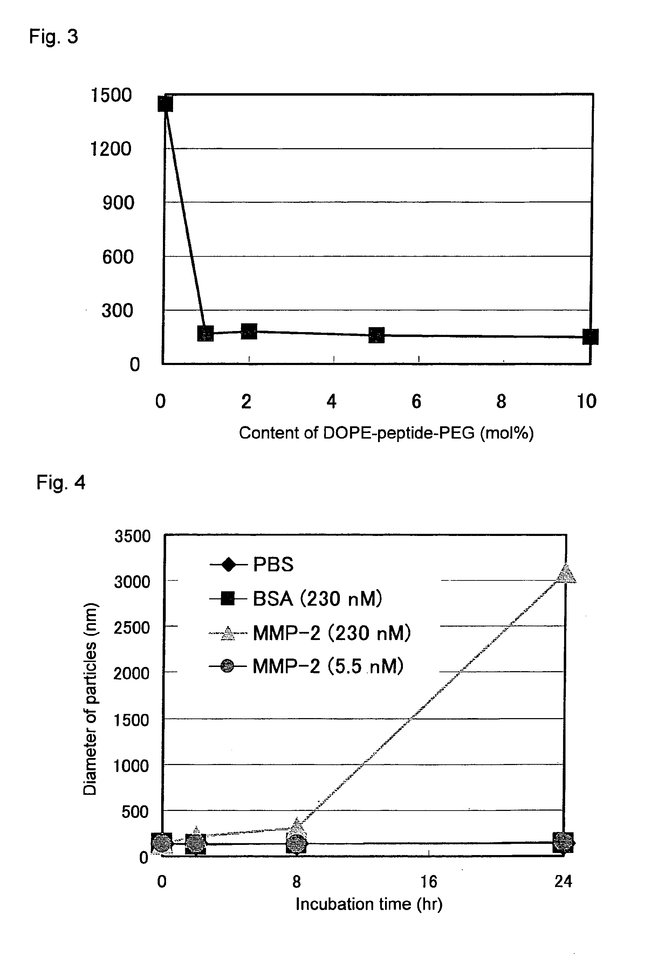

The relationship between the addition ratio of PEG-peptide-DOPE and the particle diameter is shown in Fig. 3. Also in this experiment, liposomes having a particle size less than 200 nm and showing stable dispersion were similarly prepared with the addition of 1% of PEG-peptide-DOPE. However, in this case, when the liposomes were incubated at 37°C, aggregation was observed at a PEG content up to 5%. With an addition amount of 10%, aggregation was not observed even after incubation at 37°C, and liposomes showing stable dispersion were formed. These results suggest that PEG-peptide-DOPE did not elevate the phase transition temperature as much as DOPE-PEG. A possible cause is considered that the amino acids constituting the peptide are rather hydrophobic, and hence likely to interact with the liposome membranes.

On the basis of the aforementioned results, evaluation of cleavage of PEG by MMP-2 was performed by using DOPE liposomes containing PEG-peptide-DOPE at a ratio of 10%, which did not cause aggregation even at 37°C. The results are shown in Fig. 4. Aggregation of the liposomes was not observed in PBS or with addition of 5.5 nM MMP-2, whereas aggregation was observed with addition of 230 nM MMP-2. Aggregation was not observed with the addition of 230 nM BSA, and accordingly, the aforementioned aggregation was not considered due to interaction of proteins and liposomes, but considered to be caused by the cleavage of the peptide by the action of MMP-2 to dissociate the PEG, which arose instability of the liposomes and induced the change to the inverse hexagonal structure. Form the above results, the PEG-peptide-DOPE is demonstrated to be cleaved by MMP-2 even when the lipid is in the state of constituting liposomes.

Example 3

By using D'MEM added with inactivated FBS (FBS final concentration: 10%), the human fibrosarcoma-derived HT1080 cells were cultured on a sterilized culture dish in a $CO_2$ incubator (37°C, 5% $CO_2$). When the cells reached 70% confluence, the cells were removed from the dish by using 0.5 mM EDTA-PBS, the cell suspension was diluted to a density of 1 to 5 x $10^4$ cells/mL using fresh D'MEM, and the cells were subcultured on a sterilized dish. In a similar manner, human kidney endothelium-like HEK293 cells were cultured. In the case of the HEK293 cells, the cells were removed from the dish by using 0.05% Trypsin/0.5 mM EDTA-PBS, and subcultured.

A compaction body comprising pDNA aggregated with a highly basic peptide such as PLL is formed by an electrostatic interaction between negative charge of phosphate groups of pDNA and positive charge of lysine residues, arginine residues and the like in a peptide. With definition that one phosphate group of pDNA corresponded to charge of -1, and one arginine or lysine residue corresponded to charge of +1, a charge ratio (+/-) was calculated in accordance with the following formula (1).

Formula (1):

$$\text{Charge ratio } (+/-) = \{C_P \times (n_K + n_R)/MW_P\}/(C_D/MW_D)$$

Cp: Concentration of peptide solution [mg/mL]
$n_K$: Number of lysine residues per one peptide molecule
nR: Number of arginine residues per one peptide molecule
$MW_P$: Molecular weight of peptide
$C_D$: Concentration of pDNA solution [mg/mL]
$MW_D$: Molecular weight per one nucleotide base

In order to prepare a negatively charged compaction body, the charge ratio (+/-) was determined to be 1.5 according to the formula (1). To 30 $\mu$ L of pDNA (0.5 mg/mL), 120 $\mu$L of 10 mM HEPES-HCl (pH 7.4) was added. To 5 $\mu$ L of protamine (2 mg/mL), 95 mL of 10 mM HEPES-HCl (pH 7.4) was added. Protamine was slowly added dropwise to the pDNA solution with stirring by a vortex mixer. Formation of the compaction body was confirmed by measurement of the particle diameter and zeta-potential.

Lipid solutions of 1 mM in ethanol, each of which 125 $\mu$L was taken as 100%, were put into test tubes at molar ratios of 30% DOTAP, 40% DOPE, and 30% Chol. As for addition of PEG-lipids, 1 mM PEG-DSPE solution or PEG-peptide-DOPE solution in ethanol was added at a molar ratio of 5% or 15% based on the lipids except for the PEG-lipids, which were taken as 100%. Chloroform was added in the same volume of ethanol to the mixture in each tube, and the mixture was stirred. Then, the solvent was evaporated in a desiccator to obtain a lipid membrane. Then, the compaction body solution (250$\mu$L) was added to the lipid membrane, and the mixture was left standing at room temperature for 15 to 30 minutes for hydration, and then sonicated for 1 to 2 minutes. The particle diameter and the zeta-potential were measured. The particle diameters and the zeta-potentials of the prepared MENDs are shown in Table 2. Any significant difference was not observed between the values measured for MENDs containing PEG-peptide-DOPE and MENDs containing PEG-DSPE, and thus it was considered that equivalent MENDs were successfully prepared.

[Table 2]

|  | PEG-DSPE | PEG-peptide-DOPE |
|---|---|---|
| 0% |  | 226.9 nm |
|  |  | + 47.62 mV |
| 5% | 156.3 nm | 162.2 nm |
|  | + 12.45 mV | + 18.79 mV |
| 15% | 136.7 nm | 141.2 nm |
|  | +7.55mV | + 2.80 mV |

One day before the transfection, the HT1080 cells and the HEK293 cells were each inoculated into 1 mL of a medium on a 24-well plate at a density of $4 \times 10^4$ cells/well. A sample, prepared to be corresponding to 0.4 $\mu$ g of DNA, was diluted to 250 $\mu$ L with FBS- and antibiotic-free D'MEM (hereinafter abbreviated as "D'MEM(-)") in the case of a serum free medium, or diluted similarly with D'MEM in the case of serum containing medium. Each well was washed with PBS, samples were put into wells in a volume of 250 $\mu$ L/well, and incubated at 37°C for 3 hours under 5% $CO_2$. Then, D'MEM was added in a volume of 1 mL/well, and the sample was incubated at 37°C for 45 hours under 5% $CO_2$ After the incubation for 45 hours, the medium in each well was removed, the well was washed with PBS, and Reporter Lysis Buffer was added (75 $\mu$ L/well), and frozen at -80°C. After 30 minutes, the frozen mixture on the plate was thawed at room temperature, and the cells were scraped on ice by using a cell scraper. A lysis solution was collected, and centrifuged (4°C, 15,000 rpm, 5 minutes), and the supernatant was collected. The luciferase activity (RLU) in 20$\mu$ L of the supernatant was measured, and proteins were measured according to the BCA method for the supernatant diluted 5 times with DDW to calculate RLU/mg protein.

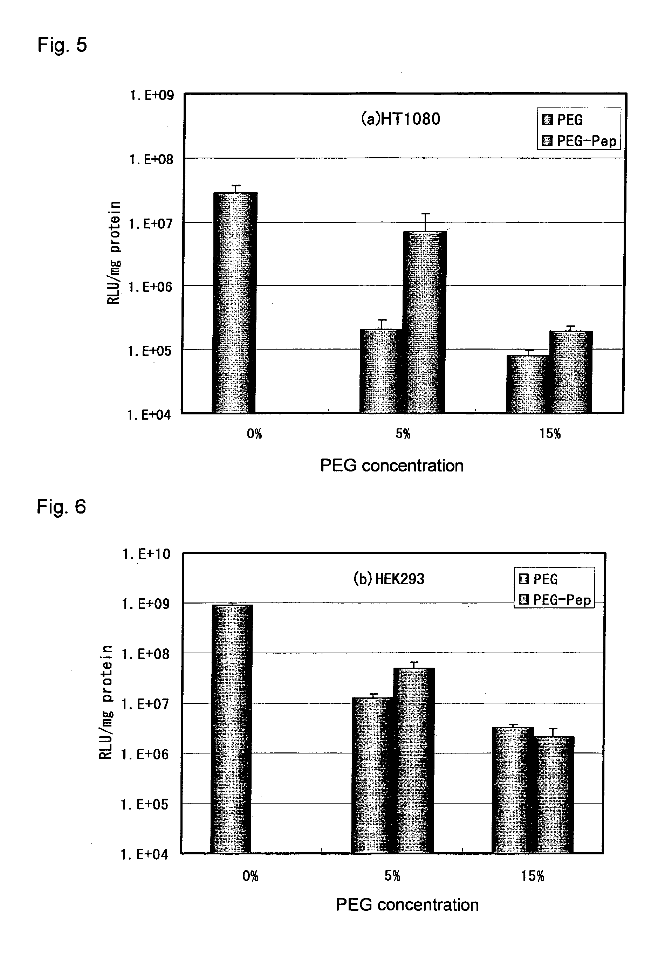

The results are shown in Figs. 5 ((a) HT1080 cells) and Fig. 6 ((b) HEK293 cells). The gene expression activity of MEND decreased in a PEG concentration-dependent manner. As for MEND modified with 15% of PEG, the PEG-peptide-DOPE modification group did not give the increase of the gene expression activity compared with the PEG-DSPE modification group for both (a) HT1080 cells and (b) HEK293 cells. As for MEND modified with 5% of PEG, gene expression was increased by PEG-peptide-DOPE 5 times in (b) HEK293 cells, and markedly increased 35 times in (a) HT1080 cells, compared with the results obtained with PEG-DSPE. Since the HT1080 cells are considered to express more MMP-2, it is considered that the above results may be dependent on the expression amount of MMP-2, and the gene expression activity was increased as a result of decomposition of the peptide by MMP-2 and cleavage of PEG. With modification with 15% of PEG, difference in the genetic expression was not observed, and this result is interpreted that significant volume of hydration layer was present due to the large amount of PEG, thereby MMP-2 failed to penetrate the hydration layers to reach the peptide moieties.

Example 4

According to the formula (1) mentioned in Example 3, the charge ratio (+/-) was determined to be 1.5, and a compaction body of pDNA coding for luciferase and protamine was prepared in HEPES buffered glucose (10 mM HEPES, pH 7.4, 5% glucose, HBG).
In the same manner as that of Example 3, a lipid membrane consisting of 30% DOTAP, 40% DOPE, and 30% Chol was prepared. To this membrane, PEG-DSPE or PEG-peptide-DOPE was added at a molar ratio of 5% or 15%, and the mixture was evaporated to obtain lipid membranes. Then, the compaction body was added to each of these lipid membranes, and the mixtures were sonicated to prepare MEND, PEG-modified MEND containing PEG-DSPE, and PEG-modified MEND containing PEG-peptide-DOPE. The particle diameters and zeta-potentials of the compaction body and the various kinds of MENDs are shown in Table 3.
[Table 3]

|  | Compaction body | MEND | PEG-DSPE | PEG-peptide-DOPE |
| --- | --- | --- | --- | --- |
| Particle diameter (nm) | 90 | 226 | 156 | 141 |
| $\zeta$-potential (mV) | -25.4 | 47.6 | 12.5 | 12.8 |

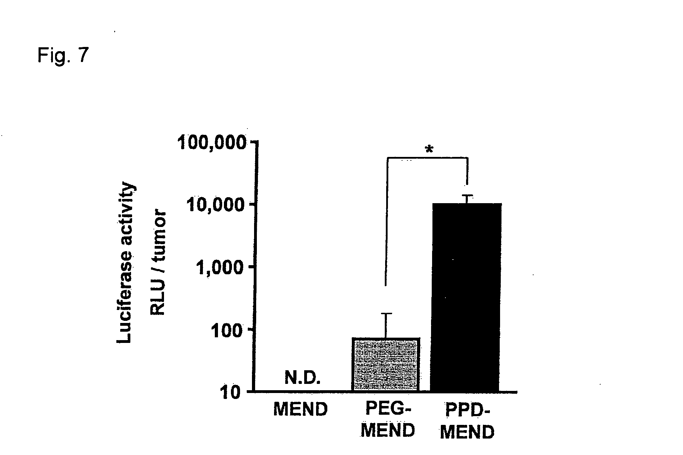

The human fibrosarcoma-derived HT1080 cells (1 x $10^6$ cells) were subcutaneously transplanted on the backs of BALB/c nude mice (male, 5-week old), when the tumor size (major axis) became 12 to 18 mm, the various kinds of MENDs (25 $\mu$ g pDNA/420 $\mu$ L in HBG, 0.5 mM lipid) were administered from the tail vein, and tumor tissues were extracted after 48 hours. Each tumor tissue was homogenized in a lysis buffer (0.1% Triton X-100, 2 mM EDTA, 0.1 M Tris-HCl, pH 7.8), the homogenate was centrifuged at 4°C and 15,000 rpm for 10 minutes, and the luciferase activity (RLU/tumor tissue) was measured for the supernatant. The results are shown in Fig. 7 (n = 3, the mean $\pm$ S.D., *P < 0.01, N.D.: not detected).
Expression of the gene was not observed with the non- PEG-modified MEND. Whilst, expression of the gene was observed with the PEG-modified MENDs, and the expression activity in tumor tissue was increased about 150 times in the PEG-peptide-DOPE modification group compared with the PEG-DSPE modification group.
It is considered that the 100 times or more of the increase in the genetic expression activity in the PEG-peptide-DOPE modification group was obtained because the peptide of PEG-peptide-DOPE was cleaved by MMP after transfer into tumor tissues to increase the gene expression activity.

Industrial Applicability

The phospholipid derivative of the present invention has a characteristic feature that the peptide moiety thereof is cleaved by a matrix metalloproteinase, and the derivative thereby dissociates the modification moiety such as poly (alkylene glycol). A lipid membrane structure such as liposome containing the phospholipid derivative of the present invention is stable in blood due to the presence of the modification moiety, whilst the lipid membrane structure becomes less stable in the vicinity of a malignant tumor cell secreting a matrix metalloproteinase because the modification moiety is dissociated. Thus the lipid membrane structure comes to be so unable not to maintain the structure and release an antitumor agent or gene retained in the lipid membrane structure into extracellular space of a malignant tumor cell, or the lipid membrane structure of which modification moiety is dissociated is efficiently taken up by the malignant tumor cell. Therefore, the agent or gene can be efficiently introduced into the malignant tumor cell.

**Claims**

1. A phospholipid derivative comprising a residue of an alcohol compound and a residue of a phospholipid, and comprising a peptide between the residue of an alcohol compound and the residue of a phospholipid, wherein

    (a) the alcohol compound is an alcohol compound selected from the group consisting of poly(alkylene glycols), glycerins, and polyglycerins,

(b) the phospholipid is a phospholipid selected from the group consisting of phosphatidylethanolamines, phosphatidylcholines, phosphatidylserines, phosphatidylinositols, phosphatidylglycerols, cardiolipins, sphingomyelins, ceramide phosphorylethanolamines, ceramide phosphorylglycerols, ceramide phosphorylglycerol phosphates, 1,2-dimyristoyl-1,2-deoxyphosphatidylcholines, plasmalogens and phosphatidic acids, and

(c) the peptide is a peptide comprising a substrate peptide that can serve as a substrate of a matrix metalloproteinase, provided that one amino acid residue or an oligopeptide containing 2 to 8 amino acid residues may bind to one or both ends of the substrate peptide.

2. The phospholipid derivative according to claim 1, wherein the alcohol compound is a poly(alkylene glycol).

3. The phospholipid derivative according to claim 1 or 2, wherein the phospholipid is a phosphatidylethanolamine.

4. The phospholipid derivative according to any one of claims 1 to 3, wherein the peptide is a peptide containing Val-Pro-Leu-Ser-Leu-Tyr-Ser-Gly.

5. The phospholipid derivative according to any one of claims 1 to 4, wherein the alcohol compound is a poly(ethylene glycol), the phospholipid is dioleoylphosphatidylethanolamine, and the peptide contains Gly-Gly-Gly as a linker.

6. The phospholipid derivative according to any one of claims 1 to 5, wherein the peptide is Gly-Gly-Gly-Val-Pro-Leu-Ser-Leu-Tyr-Ser-Gly-Gly-Gly-Gly.

7. A lipid membrane structure comprising the phospholipid derivative according to any one of claims 1 to 6 as a component lipid.

8. The lipid membrane structure according to claim 7, which is a liposome.

9. The lipid membrane structure according to claim 7 or 8, which retains an antitumor agent or a gene for gene therapy of a malignant tumor.

10. The lipid membrane structure according to claim 9, wherein the gene is a gene selected from the group consisting of antisense oligonucleotide, antisense DNA, antisense RNA, shRNA, and siRNA involved in angiogenesis or cell proliferation in malignant tumor, and a gene coding for a physiologically active substance including enzymes and cytokines, antisense RNA, shRNA, or siRNA.

11. A pharmaceutical composition for therapeutic treatment of a malignant tumor, which contains the lipid membrane structure according to claim 9 or 10.

Fig. 1

Cone-shape          Cylinder-shape          Inverse cone-shape

Inverse hexagonal structure      Lamellar structure      Micellar structure

Fig. 2

Content of DOPE-PEG (mol%)

## Fig. 3

X-axis: Content of DOPE-peptide-PEG (mol%)

## Fig. 4

Legend:
- PBS
- BSA (230 nM)
- MMP-2 (230 nM)
- MMP-2 (5.5 nM)

Y-axis: Diameter of particles (nm)
X-axis: Incubation time (hr)

Fig. 5

Fig. 6

Fig. 7

| INTERNATIONAL SEARCH REPORT | International application No. |
|---|---|
| | PCT/JP2006/303368 |

A.   CLASSIFICATION OF SUBJECT MATTER
*C07K7/00*(2006.01), *A61K47/10*(2006.01), *A61K9/127*(2006.01), *A61K31/711*
(2006.01), *A61K45/00*(2006.01), *A61K47/24*(2006.01), *A61K47/42*(2006.01),
*A61K48/00*(2006.01), *A61P35/00*(2006.01)
According to International Patent Classification (IPC) or to both national classification and IPC

B.   FIELDS SEARCHED

Minimum documentation searched (classification system followed by classification symbols)
*C07K7/00*(2006.01), *A61K47/10*(2006.01), *A61K9/127*(2006.01), *A61K31/711*
(2006.01), *A61K45/00*(2006.01), *A61K47/24*(2006.01), *A61K47/42*(2006.01),
*A61K48/00*(2006.01)

Documentation searched other than minimum documentation to the extent that such documents are included in the fields searched

Electronic data base consulted during the international search (name of data base and, where practicable, search terms used)
CA(STN), REGISTRY(STN), WPIDS(STN)

C.   DOCUMENTS CONSIDERED TO BE RELEVANT

| Category* | Citation of document, with indication, where appropriate, of the relevant passages | Relevant to claim No. |
|---|---|---|
| X,Y | JP 2003-513009 A  (The Liposome Co., Inc.), 08 April, 2003 (08.04.03), Claims; Par. Nos. [0016], [0021], [0042] & WO 01/000247 A | 1-11 |
| Y | Zhang, Janny X.; Zalipsky, Samuel; Mullah, Nasreen; Pechar, Michal; Allen, Theresa M., Pharmaco attributes of dioleoylphosphatidylethanolamine/ cholesterylhemisuccinate liposomes containing different types of cleavable lipopolymers, Pharmacological Research (2004), 49(2), 185-198, Fig.2(A), Table 1 | 1-11 |

☐   Further documents are listed in the continuation of Box C.      ☐   See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 March, 2006 (17.03.06) | 04 April, 2006 (04.04.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

<table>
<tr><td>**INTERNATIONAL SEARCH REPORT**</td><td>International application No.<br>PCT/JP2006/303368</td></tr>
</table>

(Subject to be searched)

The compound of "phospholipid derivative" of claims include a significantly wide variety of compounds in terms of the points that the peptide is not specified by a chamical structure and that what functional group the peptide and an alcohol compound or phospholipid are bonded and how they are bonded are not specified. However, only small part of the claimed compound are supported in the meaning within PCT Article 6 by the description and disclosed in the meaning within PCT Article 5 in the description.

Accordingly, claims 1-11 and description do not comply with a given requirement in such a manner as to allow a meaningful International Search.

Thus, in this International Search Report, the search was made only on prior art documents based on the compounds specifically described in the description.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003513009 A

### Non-patent literature cited in the description

- *Biochim. Biophys. Acta,* 1991, vol. 1066, 29-36
- *FEBS Lett.,* 1990, vol. 268, 235-237
- *Biochim. Biophys. Acta,* 1990, vol. 1029, 91-97
- *Cancer Res.,* 1986, vol. 46, 6387-6392
- *Cell,* 1997, vol. 91, 439-442
- *APMIS,* 1999, vol. 107, 137-143
- *Nature Biotechnology,* 2001, vol. 19, 661-667
- Molecular mechanism of cancer metastasis. Medical View Co., Ltd, 1993, 92-107
- **P.G.M. WUTS ; T. GREEN.** Protective groups in organic synthesis. Wiley, John & Sons, 1999